# EUROPEAN PATENT SPECIFICATION

(11) **EP 3 835 426 B1**
(45) Date of publication and mention of the grant of the patent: **29.06.2022**
(21) Application number: 19216155.2
(22) Date of filing: 13.12.2019
(51) Int. Cl.: C12P 7/06, C12P 19/12, C12P 19/16, C12P 19/02, C12N 9/24

(54) **PROCESS FOR THE PREPARATION OF A FERMENTABLE SUGAR COMPOSITION AND THE FERMENTATION THEREOF**
VERFAHREN ZUR HERSTELLUNG EINER FERMENTIERBAREN ZUCKERZUSAMMENSETZUNG UND DEREN FERMENTATION
PROCÉDÉ DE PRÉPARATION D'UNE COMPOSITION DE SUCRE FERMENTABLE ET SA FERMENTATION

(43) Date of publication of application: 16.06.2021
(73) Proprietor: Weissbiotech GmbH, 59387 Ascheberg (DE); BRAIN Biotech AG, 64673 Zwingenberg (DE)
(72) Inventor: de Bie, Johannes, 77600 Bussy-Saint-Georges (FR); Dirks-Hofmeister, Mareike, 69514 Laudenbach (DE); Pelzer, Alexander, 64404 Bickenbach (DE); Klermund, Ludwig, 64646 Heppenheim (DE)
(74) Representative: EDP Patent Attorneys B.V.

(56) References cited:
- WO-A1-2017/106739
- US-B2- 9 127 287
- K. POKUSAEVA ET AL: "Characterization of Two Novel alpha-Glucosidases from Bifidobacterium breve UCC2003", APPLIED AND ENVIRONMENTAL MICROBIOLOGY, vol. 75, no. 4, 29 December 2008 (2008-12-29), pages 1135-1143, XP055188644, ISSN: 0099-2240, DOI: 10.1128/AEM.02391-08

## Description

### FIELD OF THE INVENTION

The invention relates to a process for the preparation of a fermentable sugar composition and the fermentation thereof.

### BACKGROUND OF THE INVENTION

Starch is one of the important polysaccharide sources of fermentable sugars. During starch processing, starch is hydrolysed to dextrins and further to fermentable sugars like glucose or maltose. Enzymatic aids for those hydrolytic processes are α-amylases (a-1,4-glucan-4-glucanohydrolases, E.C. 3.2.1.1) to degrade high-molecular starch comprising amylose and amylopectin to liquified polysaccharide comprising lower molecular weight dextrins (liquefaction process) and glucoamylase (1,4-a-D-glucan glucohydrolase, E.C. 3.2.1.3) to cleave the a-1,4 glycosidic linkages of those dextrins releasing glucose (saccharification process).

Such fermentable sugars are of high relevance for the food sector and can be further processed e.g. by yeast fermentation to bioethanol.

### SUMMARY OF THE INVENTION

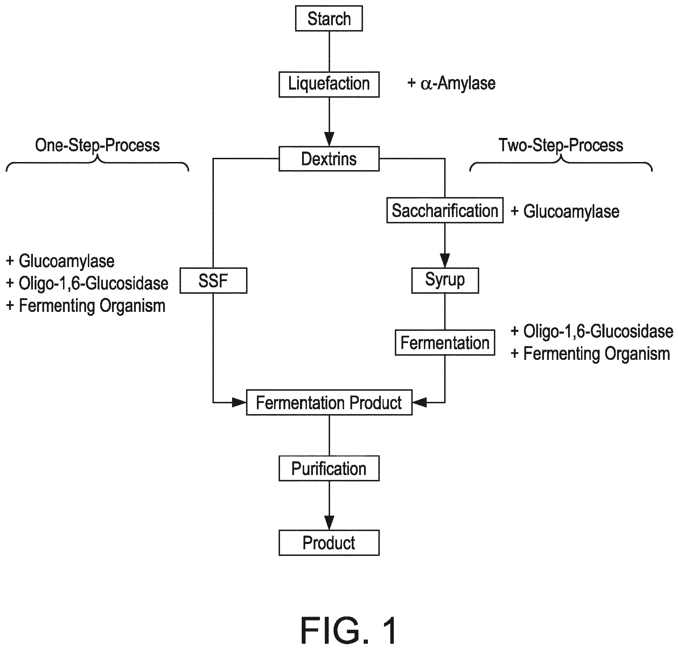

Fermentation of starch can be achieved either by performing the saccharification and fermentation in subsequent or separate processes, or by combining the two steps into one single SSF-process standing for Simultaneous Saccharification and Fermentation (Figure 1).

In an economically ideal case, the utilized liquefied polysaccharide substrate should be converted almost completely to the fermentation product with as little loss as possible. However, such losses regularly occur by the unwanted accumulation of non-fermentable sugars and thus give rise to economical and technical problems. Those non-fermentable sugars are oligosaccharides that are not linked via α-1,4-glycosidic linkages and are typically not accessible in the fermentation process.

The origins of these unwanted glycosides are (i) from the polysaccharide source by leftover a-1,6-linkages of amylopectin (a-limit dextrins), (ii) from the amylase enzyme as unwanted side-products after pro-longed liquefaction, (iii) from side-activities of the glucoamylase used in saccharification or (iv) from the metabolisms of the respective fermenting organisms. As a result, non a-1,4-linked glycosides, comprising panose (α-D-Glc-[1→6]-α-D-Glc-[1→4]-D-Glc), isomaltotriose (α-D-Glc-[1→6]-D-Glc-[1→6]-D-Glc), isomaltose (α-D-Glc-[1→6]-α-D-Glc) and/or trehalose (α-D-Glc-[1→1]-α-D-Glc), accumulate during the fermentation process. These accumulated non-fermentable sugars can constitute up to 3% of the total glycosides during the fermentation process. Hence there is a pressing need for a process utilizing alpha-1,4-linked glycosides as well as alpha-1,6-linked glycosides during a fermentation or SSF. This can be achieved by the method of the present invention.

It is an aspect of the present invention to utilize these non-fermentable sugars and thus transfer them from an economical loss into an increased yield.

It is a further aspect of the invention to use oligo-1,6-glucosidases (oligosaccharide a-1,6-glucosidase, E.C. 3.2.1.10) as a solution to the problems in the fermentation or SSF process described above.

It is a further aspect of the present invention to provide a process for the preparation of a fermentable sugar composition, wherein a polysaccharide composition is treated by at least one protein having glucoamylase enzymatic activity and at least one protein having oligo-1,6-glucosidase enzymatic activity, and wherein fermentable sugars are (substantially) removed during the treatment with the protein having oligo-1,6-glucosidase enzymatic activity.

### BRIEF DESCRIPTION OF THE DRAWINGS

Figure 1: Scheme on processing starch to gain fermentation products. Starch is first treated with α-amylase enzymes for liquefaction to gain dextrins. Those are then either processed in an SSF (simultaneous saccharification and fermentation) containing mixtures of glucoamylase enzyme, an oligo-1,6-glucosidase as described in this invention and a fermenting organism, to yield a fermentation product. Alternatively, a separated process would first saccharify the dextrins with a glucoamylase enzyme to a syrup, which is subsequently used as substrate for a fermentation step in which an oligo-1,6-glucosidase enzyme is applied.

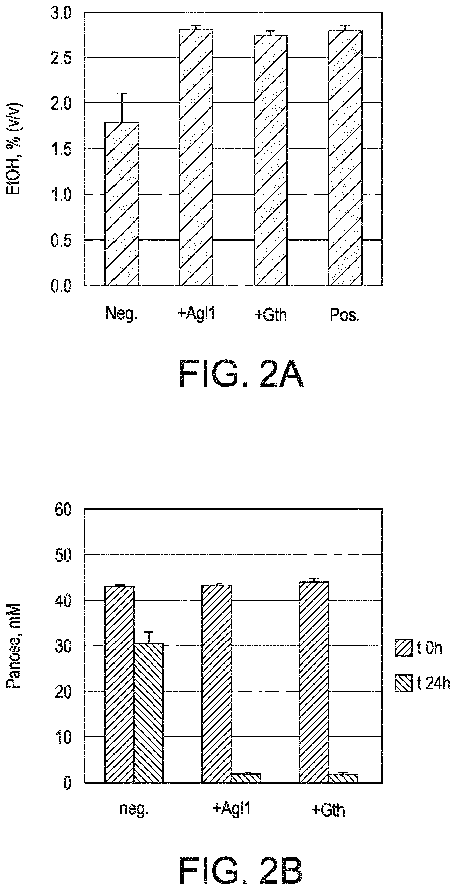

Figure 2: Shows ethanol production (A) and degradation of panose (B) in SSF after 24 h. Samples contained maltodextrins and additional 2% panose. Negative control (neg.) contains glucoamylase only, +Agl1 and +Gth contain glucoamylase plus 187.5 kU per ton of sugar/starch equivalent of said oligo-1,6-glucosidase Agll or Gth, respectively. Positive control (pos.) contains additional 2 % glucose instead of the panose and glucoamylase only.

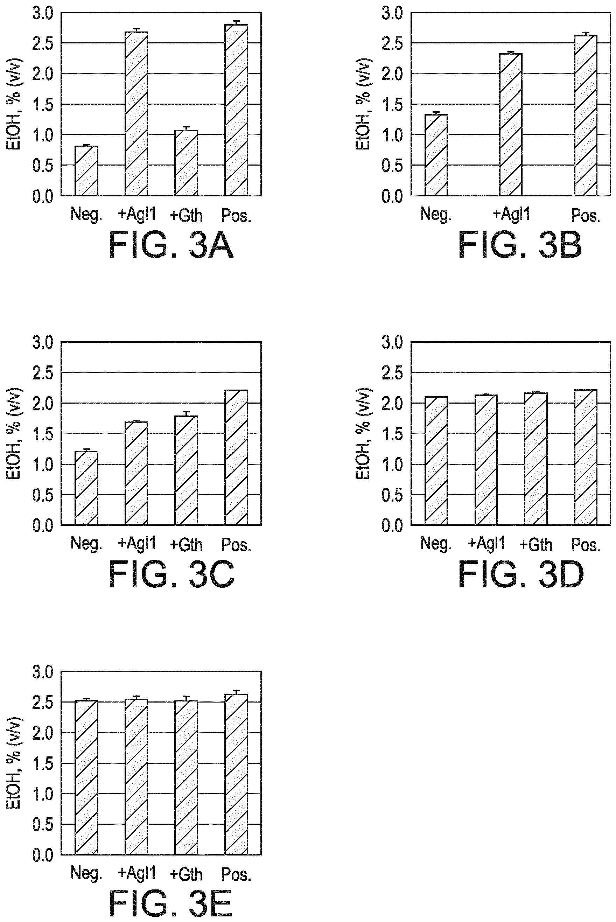

Figure 3: Shows ethanol production in SSF after 24 h from maltodextrins with additional 2% trehalose (A), or 2% isomaltose (B), or 2 % isomaltotriose (C), or 2 % maltose (D) or 2 % maltotriose (E). Negative control (neg.) contains glucoamylase only, +Agl1 and +Gth contain glucoamylase plus 187.5 kU per ton of sugar/starch equivalent of said oligo-1,6-glucosidase Agll or Gth, respectively. In (A) 937.5 kU per ton of sugar/starch equivalent of said oligo-1,6-glucosidase Agll or Gth, respectively, were used. Positive control (pos.) contains additional 2 % glucose instead of the respective sugar and glucoamylase only.

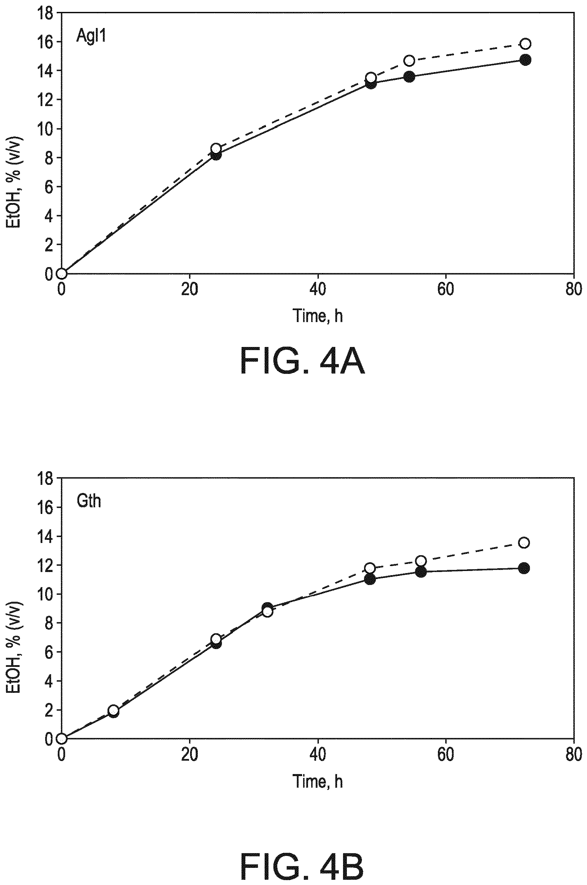

Figure 4: Illustrates the course of ethanol concentration during SSF when comparing experiments with (o) or without (•) addition of a 187.5 kU per ton of sugar/starch equivalent of oligo-1,6-glucosidase (Agl1 or Gth). Samples contained maltodextrins and additional 2% panose.

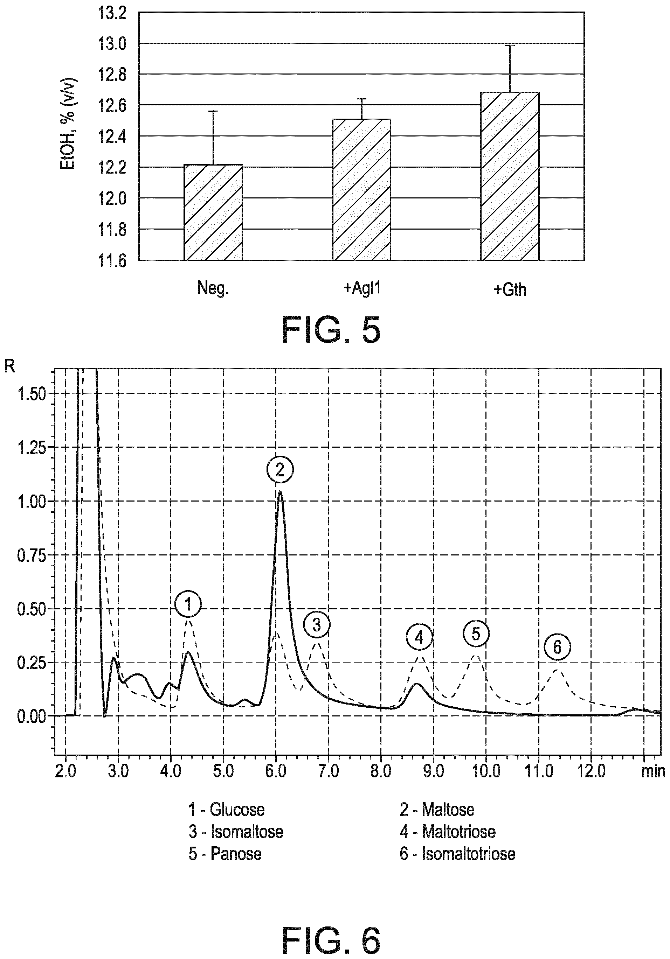

Figure 5: Shows the ethanol production in SSF with liquefied starch after 72 h. Negative control (neg.) contains glucoamylase only, +Agl1 and +Gth contain glucoamylase plus 187.5 kU per ton of sugar/starch equivalent of said oligo-1,6-glucosidase Agll or Gth, respectively
Figure 6: Shows overlay of HPLC chromatograms of the liquefied starch prior to SSF (black) and a standard (dotted) containing 5 g/L of glucose①, maltose②, isomaltose③, maltotriose④, panose⑤, isomaltotriose⑥. The liquefied starch contains no detectable non-fermentable sugars. R means refractive index (of HPLC efflux).

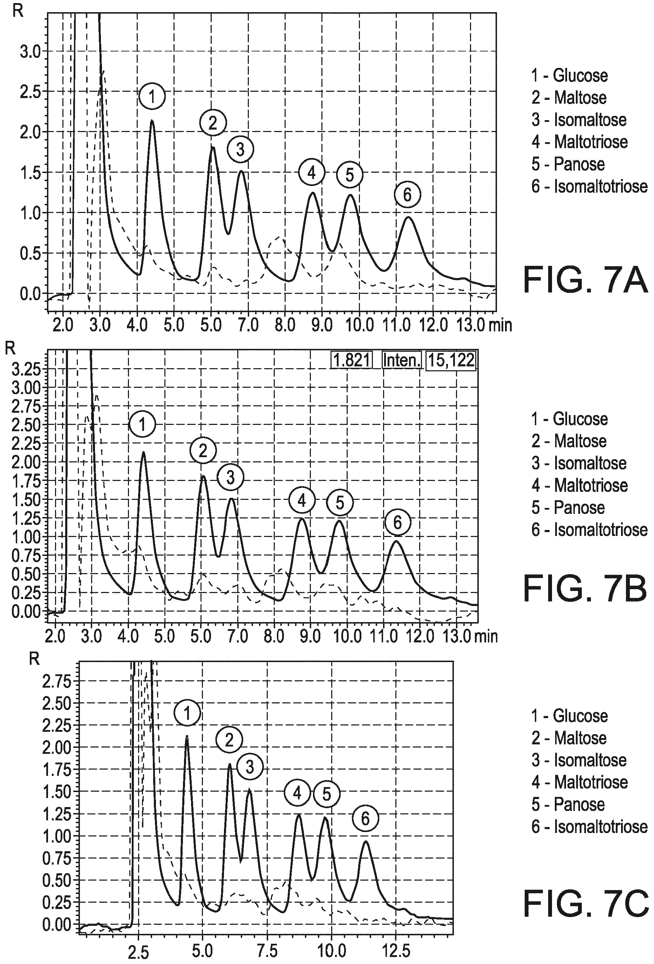

Figure 7: Shows HPLC chromatograms (dotted lines) showing residual sugars after SSF without oligo-1,6-glucosidase (A), with Agll (B), and with Gth (C) and in comparison a standard (black) containing 5 g/L of glucose①, maltose②, maltotriose④, panose⑤, isomaltose③, isomaltotriose⑥. After SSF, no non-fermentable sugars (panose, isomaltose, isomaltotriose, trehalose) were detected. R means refractive index (of HPLC efflux).

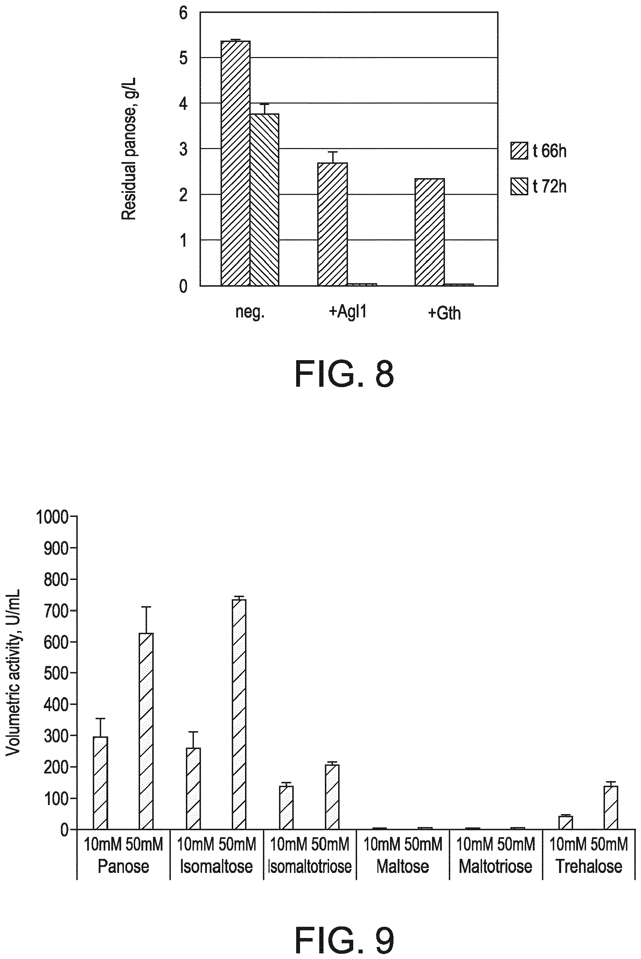

Figure 8: Shows panose concentration during SSF with liquefied starch measured after 66 h and 72 h. Negative control (neg.) contains glucoamylase only, +Agl1 and +Gth contain glucoamylase plus 187.5 kU per ton of sugar/starch equivalent of said oligo-1,6-glucosidase Agll or Gth, respectively.
Figure 9: Illustrates the biochemical characterization of Agl1. Activity is shown for different sugars at 10 mM and 50 mM. Herein, the term "Units" (U) refers to the amount of enzyme required to produce one µmol glucose per minute. Error bars are standard deviations of at least triplicates.

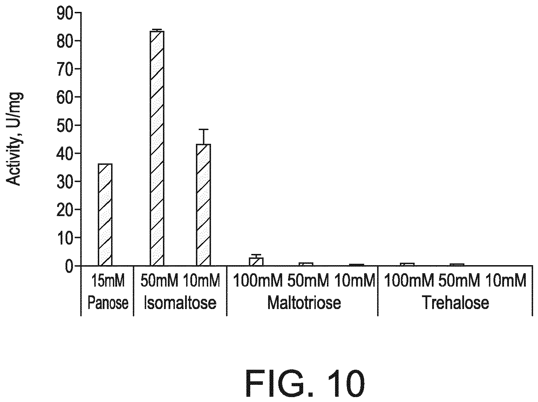

Figure 10: Illustrates the biochemical characterization of Gth. Activity is shown for different sugars at 100 mM, 50 mM, or 10 mM and for panose at 15 mM. Units refers to the amount of enzyme required to produce one µmol glucose per minute. Error bars are standard deviations of at least triplicates.

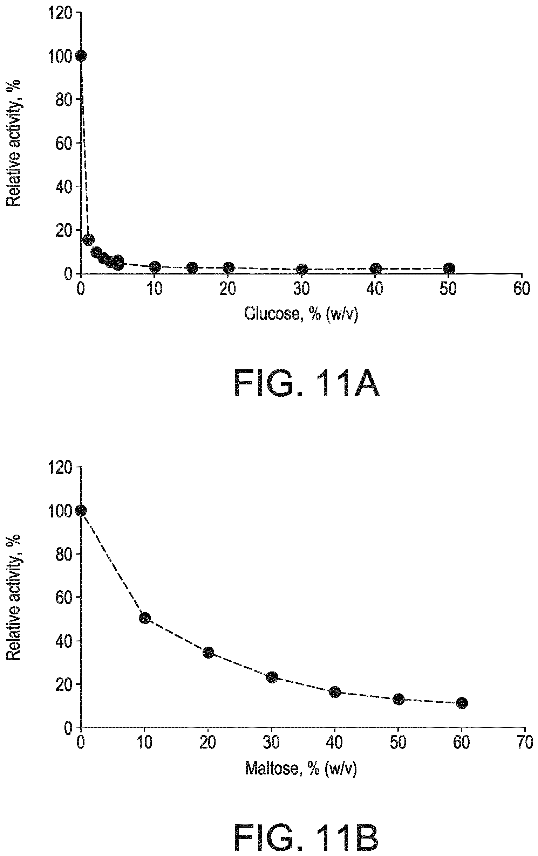

Figure 11: Illustrates the product inhibition of Agll by various glucose concentrations (A) and various maltose concentrations (B). The residual activity of Agll at various product concentrations is relative to the activity without additional product.

### DETAILED DESCRIPTION OF THE EMBODIMENTS

In one aspect the invention relates to an SSF process for the preparation of a fermentable sugar composition, wherein a polysaccharide composition is treated by an enzyme mixture of at least one protein having glucoamylase enzymatic activity and at least one protein having oligo-1,6-glucosidase enzymatic activity, and wherein the fermentable sugars are (substantially) removed during the process, wherein the protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

As indicated above, the fermentable sugars are (substantially) removed during the treatment with the protein having oligo-1,6-glucosidase enzymatic activity. The removal may especially be done in order to prevent inhibition of the enzymes with oligo-1,6-glucosidase activity. Hence, during the process for the preparation of a fermentable sugar composition the fermentable sugars are removed. Therefore, there may essentially be no detectable amounts of fermentable sugars.

According to a further aspect of the invention the polysaccharide composition is first treated by at least one protein having glucoamylase enzymatic activity and the resulting composition is subsequently treated with at least one protein having oligo-1,6-glucosidase enzymatic activity and wherein the fermentable sugars are (substantially) removed during the treatment with the protein having oligo-1,6-glucosidase enzymatic activity, wherein the protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

According to the present invention the fermentable sugars are removed by consumption thereof in a fermentation process.

In the present context with "fermentation process" we mean a metabolic process that changes the biochemical composition of an organic medium through the action of metabolising microorganisms. During said process parts of the organic medium (e.g. sugars, proteins, nutrients) are consumed by the microorganisms to produce biomass, carbon dioxide and other fermentation products.

According to one embodiment of the present invention the fermentation process may comprise producing a fermentation product. Hence, the fermentable sugar composition may be fermented to yield a fermentation product. The fermentation product may comprise any molecule producible by a (fermenting) microorganism. In further embodiments, the fermentation product may comprise one or more of an alcohol, an organic acid, or an amino acid.

The fermentable sugar composition may be fermented to yield an alcohol. For such alcoholic fermentation, *Saccharomyces cerevisiae* is the most commonly employed yeast in industrial ethanol production, combining high ethanol productivity and tolerance (18% v/v) and growth in low pH environments (reducing bacterial contamination). However, any microorganism capable of alcoholic fermentation can be deployed, such as different yeasts (e.g. *Hanseniaspora, Candida, Cyberlindnera, Kluyveromyces, Zygosaccharomyces, Schizosaccharomyces, Torulaspora, DekkeralBrettanomyces),* bacteria *(Microbacterium, Corynebacterium, Gluconobacter, Acetobacter, Pseudomonas, Vibrio, Gliocladium, Lactobacillus, Erwinia, Bacillus, Brevibacterium, Arthrobacter)* and fungi (e.g. *Aspergillus, Penicillium, Rhizopus).*

In a process for the fermentative production of an alcohol a polysaccharide composition is treated by an enzyme mixture of at least one glucoamylase enzyme and at least one oligo-1,6-glucosidase enzyme, thereby providing a fermentable sugar composition, wherein the fermentable sugar is simultaneously fermented to yield the alcohol, wherein the protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

The fermentable sugar composition may be fermented to yield organic acids or amino acids.

Organic acids which can be prepared by fermentation are for example citric acid, gluconic acid, malic acid, lactic acid, acetic acid, L-ascorbic acid, itaconic acid, propionic acid, succinic acid and/or pyruvic acid.

Citric acid typically can be prepared by *Aspergillus* sp., *Penicillium* sp., *Candida* sp., *Ustilago* sp. or *Corynebacterium* sp.. Gluconic acid typically can be prepared by *Gluconobacter, Acetobacter, Pseudomonas, Vibrio, Aspergillus, Penicillium, Ustilago* or *Gliocladium.* Lactic acid typically can be prepared by *Lactobacillus.* Acetic acid typically can be prepared by *Gluconobacter* or *Acetobacter.* L-ascorbic acid typically can be prepared by *Acetobacter, Erwinia, Bacillus* or *Corynebacterium.* Itaconic acid and malic acid can for example be prepared by *Aspergillus* or *Ustilago.*

In a process for the fermentative production of an organic acid a polysaccharide composition is treated by an enzyme mixture of at least one glucoamylase enzyme and at least one oligo-1,6-glucosidase enzyme, thereby providing a fermentable sugar composition, wherein the fermentable sugar is simultaneously fermented to yield the organic acid, wherein the protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

Amino acids which can be prepared by fermentation are for example alanine, arginine, glutamic acid, glutamine, lysine, proline, threonine, tryptophan, valine.

Alanine can for example be prepared by *Microbacterium ammoniaphilum.* Arginine can suitably be produced by certain *Serratia marcescens* species. Glutamic acid can be produced by *Corynebacterium glutamicum, Brevibacterium flavum* or *Arthrobacter paraffineus.* Glutamine can be produced by *Corynebacterium glutamicum.* Lysine can be prepared by *Brevibacterium lactofermentum* or *Brevibacterium flavum.* Proline can be produced by *Corynebacterium acetocidophylum.* Threonine as well as tryptophan can be produced by certain *E. coli* species. Valine can be produced by certain *Brevibacterium lactofermentum* species.

In a process for the fermentative production of an amino acid a polysaccharide composition is treated by an enzyme mixture of at least one glucoamylase enzyme and at least one oligo-1,6-glucosidase enzyme, thereby providing a fermentable sugar composition, wherein the fermentable sugar is simultaneously fermented to yield the amino acid, wherein the protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

The present invention also relates to a process for the production of a fermentation product from a polysaccharide composition, wherein the polysaccharide composition is treated by at least one protein having glucoamylase enzymatic activity and at least one protein having oligo-1,6-glucosidase enzymatic activity, and wherein the resulting fermentable sugar composition is (substantially) removed during the treatment with the protein having oligo-1,6-glucosidase enzymatic activity by a fermentation process in a fermentation medium, and wherein the desired fermentation product is isolated from the fermentation medium, and wherein the protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

The fermentation product (such as an alcohol, an organic acid or an amino acid) may be isolated from the fermentation medium in an aqueous medium.

For the isolation of the fermentation product (optionally after removal of all particulate material) any method known in the art for these products may be applied, such as chromatography and (in particular for lower alcohols) distillation.

In the present context a fermentable sugar composition comprises glucose, maltose and other α-1,4-linked oligo-saccharides (maltodextrins).

The polysaccharide composition which can be used according to the present invention is any composition comprising maltodextrins or other dextrins, and can for example be a starch hydrolysate. Preferably a starch hydrolysate can be used, which can be obtained from starch by acid-treatment or enzymatic digestion e.g. with a-amylase. The starch may be derived from any of various plant sources (such as corn, wheat, potato, rice and cassava).

In the present context a protein having glucoamylase enzymatic activity may mean a native glucoamylase derived from a natural source or a variant or mutant protein having at least 50%, such as at least 55%, or at least 60%, or at least 65%, or at least 70%, or at least 75%, or at least 80%, or at least 85%, or at least 90%, or at least 95% %, or at least 98% sequence identity to a glucoamylase derived from a natural source, while still having glucoamylase enzymatic activity.

For use in the fermentation processes according to the present invention a glucoamylase of the enzyme class EC 3.2.1.3 can be selected from diverse microbiological sources, mostly moulds and yeasts but also bacteria. Examples of sources for industrial use are *Aspergillus* sp., *Rhizopus* sp. and *Bacillus* sp. It is known to hydrolyse terminal (1→4)-linked alpha-D-glucose residues successively from non-reducing ends of polysaccharide chains with release of beta-D-glucose. The systematic name of this enzyme is glucan 1,4-alpha-glucosidase. Equivalent names for this enzyme may include amyloglucosidase (AMG), gamma-amylase, lysosomal alpha-glucosidase, acid maltase, exo-1,4-alpha-glucosidase, glucose amylase, gamma-1,4-glucan glucohydrolase, acid maltase, 1,4-alpha-D-glucan glucohydrolase, 4-alpha-D-glucan glucohydrolase.

In the present context a protein having oligo-1,6-glucosidase enzymatic activity may mean a native oligo-1,6-glucosidase derived from a natural source or a variant or mutant protein having at least 50%, such as at least 55%, or at least 60%, or at least 65%, or at least 70%, or at least 75%, or at least 80%, or at least 85%, or at least 90%, or at least 95% %, or at least 98% sequence identity to a oligo-1,6-glucosidase derived from a natural source, while still having oligo-1,6-glucosidase enzymatic activity.

For use in the fermentation processes according to the present invention an oligo-1,6-glucosidase of the enzyme class EC 3.2.1.10 may be selected from diverse sources, like e.g. from *Bacillus* sp., *Bifidobacterium* sp., *Saccharomyces* sp., *Sulfolobus* sp., but also from higher organisms like human or plants. It is known to hydrolyse (1→6)-alpha-D-glucosidic linkages in oligosaccharides. The systematic name of this enzyme is oligosaccharide 6-alpha-glucohydrolase, and this enzyme is also known as oligo-1,6-glucosidase, alpha-glucosidase, dextrin 6-alpha-D-glucanohydrolase, disaccharidase, exo-oligo-1,6-glucosidase, sucrase, isomaltase, limit-dextrinase, oligosaccharide alpha-1,6-glucosidase, sucrase isomaltase.

Several oligo-1,6-glucosidase sequences are publicly available, however neither their performance under process conditions (pH 4.3-4.8, 30-35°C for fermentative alcohol production, concomitance with living microorganisms and high sugar contents) nor any effect of their use in fermentation processes were shown.

The process according to the present invention may apply an enzyme based on oligo-1,6-glucosidase Gth of *Parageobacillus thermoglucosidasius* as described in WO2009/152285 and US9127287 or on oligo-1,6-glucosidase Agll of *Bifidobacterium breve* UCC2003 as described in US7268221B2 and US7615365B2 The protein having oligo-1,6-glucosidase enzymatic activity comprises a protein having at least 90% sequence identity to the amino acid sequence SEQ ID NO:1 of oligo- 1,6-glucosidase Agll of Bifidobacterium breve UCC2003 or to the amino acid sequence SEQ ID NO:2 of oligo-1,6-glucosidase Gth of Parageobacillus thermoglucosidasius.

This successful performance of the process according to the invention is particularly surprising because (i) the amount of non-fermentable sugars is very low resulting in low enzyme activity, (ii) the content of fermentable sugars and thus products of the oligo-1,6-glucosidase reaction can be very high and thus may lead to product inhibition, and (iii) also 1,1-linked non-fermentable sugars (Trehalose) can be converted into fermentable sugar and leads to an increase in the yield of fermentation product. Most surprisingly, the effect on the sugar profile and fermentative production of the SSF surpasses that of the expected content of non-fermentable sugars. This is illustrated in Example 3 wherein we find an increase in fermentation product although the control without oligo-1,6-glucosidase has no detectable amounts of non-fermentable sugars.

Our experiments under application conditions surprisingly showed that any of the oligo-1,6-glucosidases of *Bifidobacterium breve* UCC2003 (Agll; amino acid sequence SEQ ID NO:1) and *Parageobacillus thermoglucosidasius* (Gth; amino acid sequence SEQ ID NO:2) used in cooperation with glucoamylase enzyme resulted in a substantial increase of fermentation yields.

For use according to the present invention the protein having oligo-1,6-glucosidase enzymatic activity may comprise a protein having at least 90%, or at least 95% %, or at least 98% sequence identity to the amino acid sequence SEQ ID NO:1 of oligo-1,6-glucosidase Agll of *Bifidobacterium breve* UCC2003 or to the amino acid sequence SEQ ID NO:2 of oligo-1,6-glucosidase Gth of *Parageobacillus thermoglucosidasius,* while still having oligo-1,6-glucosidase enzymatic activity.

It has been found that Agl1 and Gth addition shows a 2% increase of ethanol yield from a lab-scale SSF run with 32% dextrins supplemented with panose, 30°C at pH 4.8 for 72 h with commercial glucoamylase (415 g/t of starch Deltazym^{®} GA L-E5) and alcohol yeasts (6 g/L fermentation Deltaferm^{®} AL-18).

An enzyme used in the process according to the present invention may be obtained from the organism which naturally produces such enzyme. Alternatively, the enzyme may be produced by a host cell which is transformed to produce the enzyme or several of the enzymes needed in the process of the invention by recombinant techniques known in the art.

The enzyme may be applied during the process of the invention in the form of whole cells producing the enzyme.

The enzyme may be applied during the process of the invention in the form of a lysate of the cells producing the enzyme.

The enzyme may be applied during the process of the invention in a more or less purified form, such as in a form essentially free from particulate material from the producing cells. Purification of the enzyme may be performed by any method known in the art.

The enzyme may be applied during the process of the invention in immobilized form. Many useful methods for immobilization of enzymes are known in the art. The enzymes may be immobilized for example by covalent bonding to a suitable surface or a carrier, by adsorption to a suitable surface, by entrapment e.g. in microspheres, by crosslinking e.g. to each other and/or with a suitable matrix material, or by affinity binding.

The enzymes glucoamylase and oligo-1,6-glucosidase may be used in the process of the invention in a concentration chosen to be optimal for the enzymatic conversions, and the concentration may range between 10 and 1000 kU per ton of dry cereal, preferentially between 50 and 200 kU per ton of dry cereal.

Given the nature of the polysaccharide composition used as a starting material for the process according to the invention the process will generally take place in an aqueous process medium, preferably in water. However, the process medium may also contain any aqueous buffer, e.g. phosphate buffer, bicarbonate buffer, or any other buffer solution, or mixtures of buffer solutions, or unbuffered solutions, or containing organic solvents, e.g. ethanol, methanol, DMSO, or any organic solvent, or combinations of organic solvents.

During the process of the invention the pH of the medium can vary from 4.0 to 8.0, with an optimal range of pH 4.0 to 5.5, more specifically of pH 4.3 to 4.8, and may also be dependent on the nature and concentration of the product produced by the fermentation.

During the process of the invention the temperature may range between 20 to 70°C, with an optimal range of 30 to 50°C, more specifically of 30 to 35 °C.

It will be clear to the person skilled in the art that the process conditions may be selected based on the nature and concentration of the fermentation product and on the fermenting organism. Hence, the process conditions may especially be selected to be suitable for the fermenting organism to produce the (desired) fermentation product.

### EXAMPLES

### General methods

### Cloning and expression

For use of Agll and Gth during the processes described in the invention, the enzymes need to be applied in their enzymatic active form. A person skilled in the art will have the ability to use a suitable expression system consisting of a suitable expression host, such as a microorganism, e.g. *Bacillus subtilis,* and a suitable expression construct fitting the expression host, to produce active enzymes.

The genes of Agll and Gth were obtained from public databases (National Center for Biotechnology Information (NCBI), US) [Agl1: GenBank FJ386389.1; Gth: GenBank NZ_CP012712.1 Region: 1287657 - 1289345] and cloned into a suitable *Bacillus-expression* vector known in the art, such as derivates of pUB110 (American Type Culture Collection ATCC^{®} 37015^{™}, Zyprian & Matzura 1986) or pMA5 (Dartois *et al.* 1994), containing a promotor sequence, such as Pveg, PhpaII, PamyE or P43, upstream of the gene and a termination sequence, such as rrnB, downstream of the gene. If using such common shuttle vectors an origin of replication for propagation in *Escherichia coli* and an origin of replication for replication in *Bacillus subtilis* as well as selection markers, such as ampicillin or kanamycin, need to be present. In the present invention, such a vector was constructed and propagated in *E. coli* prior to transformation into *B. subtilis* as exemplary host for enzyme production.

For production of Agll and Gth, *Bacillus subtilis* was grown overnight in LB medium as a liquid culture. One millilitre of the liquid culture was used as inoculum for expression in 100 mL A5 low phosphate medium (1.76 g/L K₂HPO₄, 2 g/L (NH₄)₂SO₄, 3.63 g/L Na₂HPO₄, 10 g/L glucose, 1.23 g/L MgSO4^{∗}7H₂O, 10 g/L casamino acids, pH 6.8) supplemented with trace elements (50 mM FeSO₄, 10 mM MnSO₄, 10 mM ZnSO₄, 2 mM CoSO₄, 2 mM CuSO₄, 2 mM NiSO₄, 2 mM Na₂MoO₄, 2 mM Na₂SeO₃, 2 mM H₃BO₃). After 16 h constitutive expression, the cells were harvested by centrifugation at 8000xg and resuspended in 20 mM phosphate buffer, pH 7.4. The cells were broken by ultrasonication in a Branson sonifier (duty cycle 50 % output control 7-9, 2 min, up to 5 repeats). The enzyme product was either used as crude sterile-filtered *Bacillus* extract, or centrifuged at 17,000xg to remove cell debris and purified using anion exchange chromatography (XK-16 column, IEX-Q-Beads, Bio-Rad Laboratories, Inc., US) followed by size exclusion chromatography (Superdex 200 Increase 10/300 GL, GE Healthcare).

### Determination of protein amount

The amount of enzyme product (Agl1 and Gth respectively) was determined via densitometry on a standard SDS-PAGE and via activity measurements. Whole protein content and purified enzyme concentration were determined via BCA assay (Pierce, Thermo Fischer Scientific).

### Activity measurements

Glucosidase activity was quantified via a glucose assay kit (D-Glucose HK Assay Kit, Megazyme u.c. IRE). Panose was added to the enzyme product at 15 mM in 50 mM Britton-Robinson buffer, pH 5. The reaction was allowed to proceed for 10 min at 37°C. The reaction was stopped by the addition of 60 µL 1 M NaOH. The pH was neutralized by the addition of 60 µL 1 M HCl. The produced glucose was quantified using the glucose assay kit following the manufacturer's instructions. One Unit (U) refers to the amount of enzyme needed to produce 1 µmol of glucose per minute.

### Quantification of ethanol yield

Ethanol was quantified via HPLC. Samples were taken at the end of the SSF or during SSF at set time points. Samples were centrifuged for 1 min at 17,000xg and sterile filtered (0.2 µm). The samples were frozen at -80°C prior to measurements. Ethanol was determined with an Aminex 87C column (Bio-Rad Laboratories, Inc., US) on a Shimadzu HPLC system.

### Quantification of residual sugars

Residual sugars were quantified via HPLC. Samples were taken at the end of the SSF or during SSF at set time points. Samples were centrifuged for 1 min at 17 000xg and sterile filtered (0.2 µm). The samples were frozen at -80°C prior to measurements. Residual sugars were determined with a Zorbax NH2 column (Agilent Technologies, Inc., US) on an Agilent 1200 HPLC system.

### Analysis of liquefied starch

Liquefied starch was analysed prior to use on a Zorbax NH2 column (Agilent Technologies, Inc., US) for analysis of mono-, di- and trisaccharide type and content, and on an Aminex 42A column (Bio-Rad Laboratories, Inc., US) for analysis of degree of polymerisation (DP) detecting DP1-DP6 content. HPLC was performed on a Shimadzu HPLC system.

### Example 1:

### Conversion of non-fermentable sugars to ethanol to test functionality of oligo-1,6-glucosidases in SSF set-up

### Simultaneous saccharification and fermentation

For simultaneous saccharification and fermentation (abbreviated SSF), the oligo-1,6-glucosidases - either Agll or Gth - were used as crude *Bacillus* extract. Glucidex^{®} Maltodextrin 12 (Roquette, FR) was dissolved in SSF-medium (5 g/L yeast extract, 5 g/L peptone, 1 g/L K₂HPO₄, 0.5 g/L Mg₂SO₄ in 50 mM citrate buffer, pH 4.8) at 300 g/L. Panose, isomaltose, isomaltotriose, maltose, maltotriose, or trehalose was added at 20 g/L. As a positive control, 20 g/L glucose was added instead of the respective sugar. The SSF medium containing starch hydrolysate (maltodextrins) and sugars was sterile filtered (0.2 µm). Yeast (Deltaferm^{®} AL-18, WeissBioTech GmbH, Ascheberg, Germany) was grown in YINaC medium (50 g/L dextrose, 5 g/L yeast extract, 5 g/L peptone, 1 g/L K₂HPO₄, 0.5 g/L Mg₂SO₄^{∗}7H₂O, 2 g/L NH₄Cl in 50 mM sodium citrate, pH 4.8) for 2 days at 37°C and harvested by centrifugation for 15 min at 5100 rpm and 8°C. Pelleted yeast was added to the SSF-medium at a final concentration of 6 g/L. Glucoamylase (Deltazym^{®} GA L-E5, WeissBioTech GmbH, Ascheberg, Germany) was added at 40 g per ton of dry starch equivalent. The enzyme product Agll or Gth was added up to a final concentration of 187.5 kU per ton of dry starch equivalent. For the SSF with trehalose, Agll or Gth was added at 937.5 kU per ton of dry starch equivalent. As a negative control, an SSF as described above was run without oligo-1,6-glucosidase/enzyme product. The SSF was performed at 30°C without stirring or shaking for 24 h.

### Results:

With the addition of panose, ethanol concentration after 24 h increased by 1.01 percentage points (v/v, increase by 92 %) with Agll and 0.94 % percentage points (v/v, increase by 85 %) with Gth compared to the negative control (Figure 2 A). The ethanol yield was not significantly different from the positive control (-0.99 percentage points v/v; 90 %). Panose concentration was reduced by 96 % with Agl1, by 96 % with Gth and in the negative control by 29 % with glucoamylase only (Figure 2 B).

With the addition of trehalose, ethanol concentration after 24 h increased by 1.86 percentage points (v/v, increase by 226 %) with Agll and 0.26 percentage points (v/v, increase by 31 %) with Gth compared to the negative control (Figure 3 A). The ethanol yield in the positive control was 1.95 percentage points (v/v) higher compared to the negative control (increase by 238 %).

With the addition of isomaltose, ethanol concentration after 24 h increased by 0.98 percentage points (v/v, increase by 74 %) with Agl1 compared to the negative control (Figure 3 B). The ethanol yield was not significantly different from the positive control.

With the addition of isomaltotriose, ethanol concentration after 24 h increased by 0.48 percentage points (v/v, increase by 40 %) with Agll and 0.57 percentage points (v/v, increase by 48 %) with Gth compared to the negative control (Figure 3 C). The ethanol yield in the positive control was 1.00 percentage point (v/v) higher compared to the negative control (increase by 83 %).

No increase in the ethanol yield was determined when maltose or maltotriose was added (Figure 3 D, E). With the addition of maltose, the ethanol concentration after 24 h increased by 0.03 percentage points (v/v, increase by 1.5 %) with Agll and 0.06 percentage points (v/v, increase by 2.8 %) with Gth compared to the negative control. The ethanol yield in the positive control was 0.11 percentage points (v/v) higher compared to the negative control (increase by 5.0 %). With the addition of maltotriose, ethanol concentration after 24 h increased by 0.03 percentage points (v/v, increase by 1 %) with Agll and 0.00 percentage points (v/v, increase by 0 %) with Gth compared to the negative control. The ethanol yield in the positive control was 0.10 percentage points (v/v) higher compared to the negative control (increase by 4.0 %).

### Conclusion:

An increase in ethanol yield compared to the control without oligo-1,6-glucosidase was observed for the non-fermentable sugars panose, isomaltose, isomaltotriose and trehalose in an SSF set-up indicating full function of the enzyme products with regard to converting non-fermentable sugars to glucose in an SSF with glucoamylase and fermenting organism. The yields obtained by adding the fermentable sugars maltose and maltotriose are unaffected by oligo-1,6-glucosidase.

### Example 2:

### Conversion of panose to ethanol to test functionality of oligo-1,6-glucosidases in industry-relevant SSF set-up

### Simultaneous saccharification and fermentation

For the SSF, the enzyme product was used as crude *Bacillus* extract. Glucidex^{®} Maltodextrin 12 was dissolved in SSF medium (5 g/L yeast extract, 5 g/L peptone, 1 g/L K₂HPO₄, 0.5 g/L Mg₂SO₄ in 50 mM citrate buffer, pH 4.8) at 300 g/L. Panose was added at 20 g/L. As a positive control, 20 g/L glucose was added. The SSF medium containing starch and sugars was sterile filtered (0.2 µm). Yeast (Deltaferm^{®} AL-18, WeissBioTech GmbH, Ascheberg, Germany) was grown in YINaC medium (50 g/L dextrose, 5 g/L yeast extract, 5 g/L peptone, 1 g/L K₂HPO₄, 0.5 g/L Mg₂SO₄^{∗}7H₂O, 2 g/L NH₄Cl in 50 mM sodium citrate, pH 4.8) for 2 days at 37°C and harvested by centrifugation for 15 min at 5100 rpm and 8°C. Pelleted yeast was added to the SSF at a final concentration of 6 g/L. Glucoamylase (Deltazym^{®} GA L-E5, WeissBioTech GmbH, Ascheberg, Germany) was added at 400 g per ton of dry starch equivalent. The enzyme product Agl1 or Gth was added to a final concentration of 187.5 kU per ton of dry starch equivalent. As a negative control, no oligo-1,6-glucosidase/enzyme product was added. The SSF was performed at 30°C without stirring or shaking for 72 h.

### Results:

With the addition of panose, ethanol concentration after 72 h increased by 1.16 percentage points (v/v, increase by 8 %) with Agl1 (Figure 4 A) and 1.77 percentage points (v/v, increase by 15 %) with Gth (Figure 4 B) compared to the negative control. The ethanol yield was not significantly different from the positive control.

### Conclusion:

An increase in ethanol yield compared to the control without oligo-1,6-glucosidase was observed for the non-fermentable sugar panose in a SSF with a process time of 72 h with the product recommended dose of glucoamylase and fermenting organism. Although the glucoamylase is capable of hydrolysing the respective amount of panose, a marked positive effect of the oligo-1,6-glucosidases Agll and Gth on the ethanol yield was detected. The oligo-1,6-glucosidases are stable and active for 72 h at pH 4.8 and 30°C.

### Example 3:

### Ethanol yield increase from commercial liquefied starch to show functionality with industry-relevant substrate in industry-relevant process conditions

### Simultaneous saccharification and fermentation

For SSF, the enzyme product was used as crude *Bacillus* extract. Acidified liquefied starch (pH 2.0) from a commercial plant, containing no detectable amounts of panose, isomaltose, isomaltotriose or trehalose, was brought to pH 4.8. SSF medium components (5 g/L yeast extract, 5 g/L peptone, 1 g/L K₂HPO₄, 0.5 g/L Mg₂SO₄ in 50 mM citrate buffer, pH 4.8) were added to liquefied starch (300 g/L). The SSF medium containing starch was sterile filtered (0.2 µm). Yeast (Deltaferm^{®} AL-18, WeissBioTech GmbH, Ascheberg, Germany) was grown in YINaC medium (50 g/L dextrose, 5 g/L yeast extract, 5 g/L peptone, 1 g/L K₂HPO₄, 0.5 g/L Mg₂SO₄^{∗}7H₂O, 2 g/L NH₄Cl in 50 mM sodium citrate, pH 4.8) for 2 days at 37°C and harvested by centrifugation for 15 min at 5100 rpm and 8°C. Pelleted yeast was added to the SSF at a final concentration of 6 g/L. Glucoamylase (Deltazym^{®} GA L-E10, WeissBioTech GmbH, Ascheberg, Germany) was added at 200 g per ton of dry starch equivalent. The enzyme product Agll or Gth was added to a final concentration of 187.5 kU per ton of dry starch equivalent. As control, no oligo-1,6-glucosidase/enzyme product was added. The SSF was performed at 30°C without stirring or shaking for 72 h.

### Results:

Ethanol concentration after 72 h increased by 0.30 percentage points (v/v, increase by 2 %) with Agll and 0.47 percentage points (v/v, increase by 4 %) with Gth compared to the negative control without oligo-1,6-glucosidase (Figure 5). No non-fermentable sugars were detected in the liquefied starch prior to its use in SSF (Figure 6). Interestingly, no residual sugars, neither fermentable nor non-fermentable and neither in the control nor in the SSFs containing oligo-1,6-glucosidase, were detected after SSF (exemplified in the control, Figure 7). To the reader skilled in the art, a 2 % ethanol yield increase during SSF is substantial.

### Conclusion:

An increase in ethanol yield was observed when adding oligo-1,6-glucosidases to an SSF compared to the control without oligo-1,6-glucosidase after saccharification and fermentation of commercial liquefied starch under industry-relevant conditions.

Interestingly, no non-fermentable sugars were detected prior and after SSF in any of the performed SSFs. Especially, no residual sugars were detected in the SSF without oligo-1,6-glucosidases, demonstrating that the addition of the enzyme product has a positive effect on the SSF even without detectable residual sugars after SSF.

### Example 4:

### Reduction in DP3 (panose) sugar content after SSF with commercial liquefied starch to show functionality with industry-relevant substrate in industry-relevant process conditions

### Simultaneous saccharification and fermentation

For SSF, the enzyme product was used as crude *Bacillus* extract. Acidified liquefied starch from a commercial plant, containing no detectable amounts of panose, isomaltose, isomaltotriose or trehalose, was brought to pH 4.8. SSF medium components (5 g/L yeast extract, 5 g/L peptone, 1 g/L K₂HPO₄, 0.5 g/L Mg₂SO₄ in 50 mM citrate buffer, pH 4.8) were added to liquefied starch (300 g/L). The SSF medium containing starch was sterile filtered (0.2 µm). Yeast (Deltaferm^{®} AL-18, WeissBioTech GmbH, Ascheberg, Germany) was grown in YINaC medium (50 g/L dextrose, 5 g/L yeast extract, 5 g/L peptone, 1 g/L K₂HPO₄, 0.5 g/L Mg₂SO₄^{∗}7H₂O, 2 g/L NH₄Cl in 50 mM sodium citrate, pH 4.8) for 2 days at 37°C and harvested by centrifugation for 15 min at 5100 rpm and 8°C. Pelleted yeast was added to the SSF at a final concentration of 6 g/L. Glucoamylase (Deltazym^{®} GA L-E10, WeissBioTech GmbH, Ascheberg, Germany) was added at 200 g per ton of dry starch equivalent. Alpha-amylase (Fuelzyme^{®}, BASF) was added at 200 g per ton of dry starch equivalent to simulate non-complete removal of alpha-amylase in industrial processes, which is often the cause for increased non-fermentable sugars. The enzyme product Agll or Gth was added to a final concentration of 187.5 kU per ton of dry starch equivalent. As control, no oligo-1,6-glucosidase/enzyme product was added. The SSF was performed at 30°C without stirring or shaking for 72 h.

### Results:

Panose was detected after 60 h in all samples. In the control SSF with no oligo-1,6-glucosidase, 5.4 g/L panose was detected after 60 h, which decreased to 3.8 g/L after 72 h (Figure 8). In the SSFs containing Agl1, 2.7 g/L panose was detected after 60 h (50 % reduction in panose compared to control) which was completely hydrolysed after 72 h. In the SSF containing Gth, 2.3 g/L panose was detected after 60 h (57 % reduction in panose compared to control), which was completely hydrolysed after 72 h.

### Conclusion:

The non-fermentable sugar panose was produced during SSF with commercial liquefied starch initially containing no non-fermentable sugars. Panose hydrolysis proceeded faster with the addition of oligo-1,6-glucosidase compared to the control containing no oligo-1,6-glucosidase and was completely hydrolysed to fermentable sugars during the process, leading to an overall reduction in DP3 residual sugars.

### Example 5:

### Biochemical characterization

### Activity assay

For activity measurements, the enzyme Agll was incubated with 10 mM and 50 mM panose, isomaltose, isomaltotriose, maltose, maltotriose, and trehalose for 10 min at 37°C in Britton-Robertson buffer, pH 5. The enzyme Gth was incubated with 15 mM panose, 10 and 50 mM isomaltose, and 10, 50 and 100 mM maltotriose and trehalose for 10 min at 37°C in Britton-Robertson buffer, pH 5.

The reaction was stopped with 60 µL of 1 M NaOH, followed by addition of 60 µL of 1 M HCl.

The amount of produced glucose was quantified using a standardized glucose assay kit (D-Glucose HK Assay Kit, Megazyme u.c. IRE).

Glucose concentration was determined as specified in the instructions. Units are defined as the amount of enzyme required to produce one µmol of glucose per minute.

The pH range was investigated from pH 3 to pH 9 by adjusting the Britton-Robinson buffer to the respective pH.

The temperature range was investigated from 30°C - 80°C by incubating the enzymes and measuring their activity at the respective temperature.

Temperature stability was determined by pre-incubating Agll at 50°C and Gth at 80°C and measuring the time required to reach 50% of the initial activity.

Process stability was determined by incubating the enzymes under process conditions (SSF medium without starch, pH 4.8, 30°C) for 72 h and measuring the remaining activity.

Storage stability was determined by measuring the activity of the enzymes stored in a glass container in a stabilizing formulation (50% v/v glycerol) at 4°C over a time period of 12 months. The storage stability was extrapolated from these data. "Without a loss in activity" is defined as an extrapolated activity of greater than 95% of the initial activity.

Product inhibition was determined by measuring hydrolysis of p-nitrophenyl-α-D-glucopyranoside (pNPG, Megazyme u.c., Ireland) by oligo-1,6-glucosidase activity in the presence of glucose or maltose from 0 - 50% (w/v). pNPG was quantified photometrically at 405 nm. Product inhibition is defined as IC50, which describes the amount of inhibitor required to reduce the enzyme activity by 50% at 2mM pNPG.

### Results:

Activity measurements confirmed degradation of the oligosaccharides panose, isomaltose and isomaltotriose (Figure 9, Figure 10). Agll additionally breaks down trehalose. In the case of panose, one equivalent of glucose and one equivalent of maltose is produced. For isomaltose, isomaltotriose and trehalose, glucose is the sole product in two, three and two equivalents.

The pH range of Agll is 4.5 - 8.0, the temperature range is from 30 - 50°C. The pH range of Gth is 4.5 - 8.0, the temperature range is from 30 - 70°C.

Agll has a thermal stability of t_{½} = 30 min at 50°C (time required for denaturation of 50 % of the enzyme), a process stability of 80 % after 72 h at 30°C and a storage stability at 4°C of 18 - 24 month without a loss in activity.

Gth has a thermal stability of t_{½} = 30 min at 80°C, a process stability of 80% after 72 h at 30°C and a storage stability at 4°C of 18 - 24 month without a loss in activity.

Product inhibition of Agl1 shows an IC50 for glucose of 0.6 % w/v or 6 g/L and for maltose of 10 % w/v or 100 g/L (Figure 11 A, B).

### Conclusion:

The enzymes Agl1 and Gth have different optimal temperature ranges with regard to activity and stability. Agll is less stable and loses 50% activity after 30 min at 50°C, whereas Gth is very stable and showed 50% loss of activity after 30 min at 80°C. Both enzymes are stable for the whole process duration of 72 h at 30°C. This is important as the high glucose and maltose concentration in the beginning of the process inhibits the enzymes with oligo-1,6-glucosidase activity. Biochemical characterization shows different substrate spectra and different substrate specificity, which do not influence performance in the invention described above.

### DEFINITIONS

As used herein the term "substantially", such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

The various aspects discussed in this patent can be combined in order to provide additional advantages.

### CITED NON-PATENT DOCUMENTS

Zyprian & Matzura 1986: Zyprian, E., Matzura, H.: Characterization of signals promoting gene expression on the Staphylococcus aureus plasmid pUB110 and development of a gram-positive expression vector system. DNA 5: 919-25 (1986).

Dartois *et al.* 1994: Dartois, V., Coppée, Y.-C., Colson, C, Baulard, A.: Genetic analysis and overexpression of lipolytic activity in Bacillus subtilis. Applied and Environmental Microbiology 60, 1670-1673 (1994).

## Claims

1. A process for the preparation of a fermentable sugar composition, wherein a polysaccharide composition is treated by at least one protein having glucoamylase enzymatic activity and at least one protein having oligo-1,6-glucosidase enzymatic activity, and wherein the fermentable sugars are removed during the treatment with the protein having oligo-1,6-glucosidase enzymatic activity, wherein the protein having oligo-1,6-glucosidase enzymatic activity comprises a protein having at least 90% sequence identity to the amino acid sequence SEQ ID NO: 1 of oligo-1,6-glucosidase Agll of *Bifidobacterium breve* UCC2003 or to the amino acid sequence SEQ ID NO:2 of oligo-1,6-glucosidase Gth of *Parageobacillus thermoglucosidasius.*

2. The process according to claim 1, wherein the polysaccharide composition is treated by a mixture of at least one protein having glucoamylase enzymatic activity and at least one protein having oligo-1,6-glucosidase enzymatic activity.

3. The process according to claim 1, wherein the polysaccharide composition is first treated by at least one protein having glucoamylase enzymatic activity and the resulting composition is subsequently treated with at least one protein having oligo-1,6-glucosidase enzymatic activity.

4. The process according to any of the preceding claims, wherein the fermentable sugars are consumed in a fermentation process.

5. The process according to claim 4, wherein the fermentable sugar composition is fermented to yield an alcohol.

6. The process according to claim 4, wherein the fermentable sugar composition is fermented to yield an organic acid, such as citric acid, gluconic acid, lactic acid, acetic acid, L-ascorbic acid, itaconic acid, propionic acid, succinic acid and/or pyruvic acid, or an amino acid.

7. The process according to any of the preceding claims, wherein the polysaccharide composition is a starch hydrolysate.

8. The process according to any of the preceding claims, wherein the protein having glucoamylase enzymatic activity is a glucoamylase enzyme belonging to the enzyme class EC 3.2.1.3.

9. The process according to any of the preceding claims, wherein the protein having oligo-1,6-glucosidase enzymatic activity is an oligo-1,6-glucosidase enzyme belonging to the enzyme class EC 3.2.1.10.

10. The process according to any of the preceding claims, wherein a protein having oligo-1,6-glucosidase enzymatic activity from *Bifidobacterium breve* UCC2003 (Agl1) and/or from *Parageobacillus thermoglucosidasius* (Gth) is used.

11. The process according to any of the preceding claims, wherein the treatment by the enzyme mixture takes place in an aqueous process medium.

12. The process according to any of the preceding claims, wherein the treatment by the enzyme mixture takes place at an acidic pH in the range of 4.0 to 8.0, with an optimal range of pH 4.0 to 5.5, more specifically at pH 4.3 to 4.8.

13. The process according to any of the preceding claims, wherein the treatment by the enzyme mixture takes place at a temperature in the range of 30 to 70°C, with an optimal range of 30 to 50°C, more specifically of 30 to 35 °C.

14. A process for the production of a fermentation product from a polysaccharide composition, wherein the polysaccharide composition is treated according to the process of any one of the preceding claims, and subsequently a fermentation process is carried out in a fermentation medium, and wherein the desired fermentation product is isolated from the fermentation medium.

15. The process according to claim 14, wherein the fermentation product is selected from an alcohol, an organic acid, and an amino acid.

## Patentansprüche

1. Verfahren zum Herstellen einer fermentierbaren Zuckerzusammensetzung, wobei eine Polysaccharidzusammensetzung mit mindestens einem Protein mit enzymatischer Glucoamylaseaktivität und mindestens einem Protein mit enzymatischer Oligo-1,6-Glucosidaseaktivität behandelt wird, und wobei die fermentierbaren Zucker während der Behandlung mit dem Protein mit enzymatischer Oligo-1,6-Glucosidaseaktivität entfernt werden, wobei das Protein mit enzymatischer Oligo-1,6-Glucosidaseaktivität ein Protein umfasst, das mindestens 90 % Sequenzidentität mit der Aminosäuresequenz SEQ ID NO: 1 der Oligo-1,6-Glucosidase Agl1 von *Bifidobacterium breve* UCC2003 oder mit der Aminosäuresequenz SEQ ID NO: 2 der Oligo-1,6-Glucosidase Gth von *Parageobacillus thermoglucosidasius* aufweist.

2. Verfahren nach Anspruch 1, wobei die Polysaccharidzusammensetzung mit einer Mischung von mindestens einem Protein mit enzymatischer Glucoamylaseaktivität und mindestens einem Protein mit enzymatischer Oligo-1,6-Glucosidaseaktivität behandelt wird.

3. Verfahren nach Anspruch 1, wobei die Polysaccharidzusammensetzung zunächst mit mindestens einem Protein mit enzymatischer Glucoamylaseaktivität behandelt wird und die resultierende Zusammensetzung anschließend mit mindestens einem Protein mit enzymatischer Oligo-1,6-Glucosidaseaktivität behandelt wird.

4. Verfahren nach einem der vorhergehenden Ansprüche, wobei die fermentierbaren Zucker in einem Fermentationsverfahren verbraucht werden.

5. Verfahren nach Anspruch 4, wobei die fermentierbare Zuckerzusammensetzung fermentiert wird, um einen Alkohol zu ergeben.

6. Verfahren nach Anspruch 4, wobei die fermentierbare Zuckerzusammensetzung fermentiert wird, um eine organische Säure, wie etwa Citronensäure, Gluconsäure, Milchsäure, Essigsäure, L-Ascorbinsäure, Itaconsäure, Propionsäure, Bernsteinsäure und/oder Brenztraubensäure, oder eine Aminosäure zu ergeben.

7. Verfahren nach einem der vorhergehenden Ansprüche, wobei die Polysaccharidzusammensetzung ein Stärkehydrolysat ist.

8. Verfahren nach einem der vorhergehenden Ansprüche, wobei das Protein mit enzymatischer Glucoamylaseaktivität ein Glucoamylaseenzym ist, das zur Enzymklasse EC 3.2.1.3 gehört.

9. Verfahren nach einem der vorhergehenden Ansprüche, wobei das Protein mit enzymatischer Oligo-1,6-Glucosidaseaktivität ein Oligo-1,6-Glucosidaseaktivität ist, das zur Enzymklasse EC 3.2.1.10 gehört.

10. Verfahren nach einem der vorhergehenden Ansprüche, wobei ein Protein mit Oligo-1,6-Glucosidaseenzymaktivität von *Bifidobacterium breve* UCC2003 (Agl1) und/oder von *Parageobacillus thermoglucosidasius* (Gth) verwendet wird.

11. Verfahren nach einem der vorhergehenden Ansprüche, wobei die Behandlung mit dem Enzymgemisch in einem wässrigen Prozessmedium erfolgt.

12. Verfahren nach einem der vorhergehenden Ansprüche, wobei die Behandlung mit dem Enzymgemisch in bei einem sauren pH-Wert im Bereich von 4,0 bis 8,0 stattfindet, wobei der optimale pH-Wert im Bereich von 4,0 bis 5,5 liegt, insbesondere im Bereich von 4,3 bis 4,8.

13. Verfahren nach einem der vorhergehenden Ansprüche, wobei die Behandlung mit dem Enzymgemisch bei einer Temperatur im Bereich von 30 bis 70 °C stattfindet, wobei die optimale Temperatur im Bereich von 30 bis 50 °C liegt, insbesondere im Bereich von 30 bis 35 °C.

14. Verfahren zum Herstellen eines Fermentationsprodukts aus einer Polysaccharidzusammensetzung, wobei die Polysaccharidzusammensetzung gemäß dem Verfahren nach einem der vorhergehenden Ansprüche behandelt wird und anschließend ein Fermentationsverfahren in einem Fermentationsmedium durchgeführt wird, und wobei das gewünschte Fermentationsprodukt aus dem Fermentationsmedium isoliert wird.

15. Verfahren nach Anspruch 14, wobei das Fermentationsprodukt ausgewählt ist aus einem Alkohol, einer organischen Säure und einer Aminosäure.

## Revendications

1. Procédé pour la préparation d'une composition de sucre fermentable, une composition de polysaccharide étant traitée par au moins une protéine possédant une activité enzymatique de glucoamylase et au moins une protéine possédant une activité enzymatique d'oligo-1,6-glucosidase, et les sucres fermentables étant éliminés pendant le traitement avec la protéine possédant une activité enzymatique d'oligo-1,6-glucosidase, et la protéine possédant une activité enzymatique d'oligo-1,6-glucosidase comprenant une protéine possédant au moins 90 % d'identité de séquence avec la séquence d'acides aminés SEQ ID NO: 1 d'oligo-1,6-glucosidase Agll de *Bifidobacterium breve* UCC2003 ou avec la séquence d'acides aminés SEQ ID NO: 2 d'oligo-1,6-glucosidase Gth de *Parageobacillus thermoglucosidasius.*

2. Procédé selon la revendication 1, la composition de polysaccharide étant traitée par un mélange d'au moins une protéine possédant une activité enzymatique de glucoamylase et au moins une protéine possédant une activité enzymatique d'oligo-1,6-glucosidase.

3. Procédé selon la revendication 1, la composition de polysaccharide étant d'abord traitée par au moins une protéine possédant une activité enzymatique de glucoamylase et la composition résultante étant subséquemment traitée avec au moins une protéine possédant une activité enzymatique d'oligo-1,6-glucosidase.

4. Procédé selon l'une quelconque des revendications précédentes, les sucres fermentables étant consommés dans un procédé de fermentation.

5. Procédé selon la revendication 4, la composition de sucre fermentable étant fermentée pour produire un alcool.

6. Procédé selon la revendication 4, la composition de sucre fermentable étant fermentée pour produire un acide organique, tel que l'acide citrique, l'acide gluconique, l'acide lactique, l'acide acétique, acide L-ascorbique, l'acide itaconique, l'acide propionique, l'acide succinique et/ou l'acide pyruvique ou un acide aminé.

7. Procédé selon l'une quelconque des revendications précédentes, la composition de polysaccharide étant un hydrolysat d'amidon.

8. Procédé selon l'une quelconque des revendications précédentes, la protéine possédant une activité enzymatique de glucoamylase étant une enzyme de type glucoamylase appartenant à la classe d'enzymes EC 3.2.1.3.

9. Procédé selon l'une quelconque des revendications précédentes, la protéine possédant une activité enzymatique d'oligo-1,6-glucosidase étant une enzyme de type oligo-1,6-glucosidase appartenant à la classe d'enzymes EC 3.2.1.10.

10. Procédé selon l'une quelconque des revendications précédentes, une protéine possédant une activité enzymatique d'oligo-1,6-glucosidase provenant de *Bifidobacterium breve* UCC2003 (Agl1) et/ou de *Parageobacillus thermoglucosidasius* (Gth) étant utilisée.

11. Procédé selon l'une quelconque des revendications précédentes, le traitement par le mélange d'enzymes ayant lieu dans un milieu de procédé aqueux.

12. Procédé selon l'une quelconque des revendications précédentes, le traitement par le mélange d'enzymes ayant lieu à un pH acide dans la plage de 4,0 à 8,0, avec une plage optimale de pH de 4,0 à 5,5, plus spécifiquement à pH de 4,3 à 4,8.

13. Procédé selon l'une quelconque des revendications précédentes, le traitement par le mélange d'enzymes ayant lieu à une température dans la plage de 30 à 70 °C, avec une plage optimale de 30 à 50 °C, plus spécifiquement de 30 à 35 °C.

14. Procédé pour la production d'un produit de fermentation à partir d'une composition de polysaccharide, la composition de polysaccharide étant traitée selon le procédé selon l'une quelconque des revendications précédentes, et subséquemment un procédé de fermentation étant mis en œuvre dans un milieu de fermentation, et le produit de fermentation souhaité étant isolé du milieu de fermentation.

15. Procédé selon la revendication 14, le produit de fermentation étant choisi parmi un alcool, un acide organique et un acide aminé.


### SEQUENCE LISTING

<110> WeissBioTech GmbH
<120> Process for the preparation of a fermentable sugar composition and the
   fermentation thereof
<130> -
<160> 2
<170> BiSSAP 1.3.6
<210> 1
   <211> 607
   <212> PRT
   <213> Bifidobacterium breve UCC2003
<220>
   <223> ACM89182.1 alpha-glucosidase 1
<400> 1
   Met Met Thr Thr Phe Asn Arg Ala Ile Ile Pro Asp Ala Ile Arg Thr
   1               5                   10                  15
   Asn Gly Ala Thr Pro Asn Pro Trp Trp Ser Asn Ala Val Val Tyr Gln
               20                  25                  30
   Ile Tyr Pro Arg Ser Phe Gln Asp Thr Asn Gly Asp Gly Phe Gly Asp
           35                  40                  45
   Leu Lys Gly Ile Thr Ser Arg Leu Asp Tyr Leu Ala Asp Leu Gly Val
       50                  55                  60
   Asp Val Leu Trp Leu Ser Pro Val Tyr Lys Ser Pro Gln Asp Asp Asn
   65                  70                  75                  80
   Gly Tyr Asp Ile Ser Asp Tyr Gln Asp Ile Asp Pro Leu Phe Gly Thr
                   85                  90                  95
   Leu Asp Asp Met Asp Glu Leu Leu Ala Glu Ala His Lys Arg Gly Leu
               100                 105                 110
   Lys Val Val Met Asp Leu Val Val Asn His Thr Ser Asp Glu His Ala
           115                 120                 125
   Trp Phe Glu Ala Ser Lys Asn Lys Asp Asp Glu His Ala Asp Trp Tyr
       130                 135                 140
   Trp Trp Arg Pro Ala Arg Pro Gly Thr Thr Pro Gly Glu Pro Gly Ser
   145                 150                 155                 160
   Glu Pro Asn Gln Trp Gly Ser Tyr Phe Gly Gly Ser Ala Trp Glu Tyr
                   165                 170                 175
   Cys Pro Glu Arg Gly Glu Tyr Tyr Leu His Gln Phe Ser Lys Lys Gln
               180                 185                 190
   Pro Asp Leu Asn Trp Glu Asn Pro Ala Val Arg Arg Ala Val Tyr Asp
           195                 200                 205
   Met Met Asn Trp Trp Leu Asp Arg Gly Ile Asp Gly Phe Arg Met Asp
       210                 215                 220
   Val Ile Thr Leu Ile Ser Lys Arg Thr Asp Ala Asn Gly Arg Leu Pro
   225                 230                 235                 240
   Gly Glu Tyr Gly Ser Glu Leu Asp Asp Leu Pro Val Gly Glu Glu Gly
                   245                 250                 255
   Tyr Ser Asn Pro Asn Pro Phe Cys Ala Asp Gly Pro Arg Gln Asp Glu
               260                 265                 270
   Phe Leu Lys Glu Met Arg Arg Glu Val Phe Ala Gly Arg Glu Gly Phe
           275                 280                 285
   Leu Thr Val Gly Glu Ala Pro Gly Ile Thr Pro Val Arg Asn Glu His
       290                 295                 300
   Ile Thr Asn Pro Ala Asn Gly Glu Leu Asp Met Leu Phe Leu Phe Asp
   305                 310                 315                 320
   His Val Asp Phe Asp Cys Asp Gly Val Lys Trp Lys Pro Leu Pro Leu
                   325                 330                 335
   Asp Leu Pro Gly Phe Lys Arg Ile Met Ala Gly Tyr Gln Thr Ala Val
               340                 345                 350
   Glu Asn Val Gly Trp Ala Ser Leu Phe Thr Gly Asn His Asp Gln Pro
           355                 360                 365
   Arg Val Val Ser Arg Trp Gly Asp Asp Ser Ser Glu Glu Ser Arg Val
       370                 375                 380
   Arg Ser Ala Lys Ala Leu Gly Leu Met Leu His Met His Arg Gly Thr
   385                 390                 395                 400
   Pro Tyr Val Tyr Gln Gly Glu Glu Leu Gly Met Thr Asn Ala His Phe
                   405                 410                 415
   Thr Ser Leu Asp Gln Tyr Arg Asp Leu Glu Ser Leu Asn Ala Tyr Arg
               420                 425                 430
   Gln Arg Val Glu Glu Ala Lys Val Gln Ser Pro Glu Ser Met Leu Ala
           435                 440                 445
   Gly Ile Ala Ala Arg Gly Arg Asp Asn Ser Arg Thr Pro Met Gln Trp
       450                 455                 460
   Asp Gly Ser Ala Tyr Ala Gly Phe Thr Ala Pro Asp Ala Ala Thr Glu
   465                 470                 475                 480
   Pro Trp Ile Ser Val Asn Pro Asn His Ala Glu Ile Asn Ala Ala Gly
                   485                 490                 495
   Glu Phe Asp Asp Pro Asp Ser Val Tyr Ala Phe Tyr Lys Lys Leu Ile
               500                 505                 510
   Ala Leu Arg His Asn Ser Ser Ile Val Ala Ala Gly Glu Trp Gln Leu
           515                 520                 525
   Ile Asp Ala Asp Asp Ala His Val Tyr Ala Phe Thr Arg Thr Leu Gly
       530                 535                 540
   Asn Glu Arg Leu Leu Val Val Val Asn Leu Ser Gly Arg Thr Val Asp
   545                 550                 555                 560
   Leu Pro Arg Glu Ser Thr Glu Leu Ile Ala Gly Gly Val Thr Glu Pro
                   565                 570                 575
   Asp Ile Ile Leu Ser Thr Tyr Asp Ala Pro His Thr Val Val Ser Leu
               580                 585                 590
   Ala Asn Arg Glu Leu Asp Pro Trp Glu Ala Ala Ala Val Gln Leu
           595                 600                 605
<210> 2
   <211> 562
   <212> PRT
   <213> Parageobacillus thermoglucosidasius
<220>
   <223> WP_042386239.1 alpha-glucosidase
<400> 2
   Met Glu Arg Val Trp Trp Lys Glu Ala Val Val Tyr Gln Ile Tyr Pro
   1               5                   10                  15
   Arg Ser Phe Tyr Asp Ser Asn Gly Asp Gly Ile Gly Asp Ile Arg Gly
               20                  25                  30
   Ile Ile Ala Lys Leu Asp Tyr Leu Lys Glu Leu Gly Val Asp Val Val
           35                  40                  45
   Trp Leu Ser Pro Val Tyr Lys Ser Pro Asn Asp Asp Asn Gly Tyr Asp
       50                  55                  60
   Ile Ser Asp Tyr Arg Asp Ile Met Asp Glu Phe Gly Thr Met Ala Asp
   65                  70                  75                  80
   Trp Lys Thr Met Leu Glu Glu Met His Lys Arg Gly Ile Lys Leu Val
                   85                  90                  95
   Met Asp Leu Val Val Asn His Thr Ser Asp Glu His Pro Trp Phe Ile
               100                 105                 110
   Glu Ser Arg Lys Ser Lys Asp Asn Pro Tyr Arg Asp Tyr Tyr Ile Trp
           115                 120                 125
   Arg Pro Gly Lys Asn Gly Lys Glu Pro Asn Asn Trp Glu Ser Val Phe
       130                 135                 140
   Ser Gly Ser Ala Trp Glu Tyr Asp Glu Met Thr Gly Glu Tyr Tyr Leu
   145                 150                 155                 160
   His Leu Phe Ser Lys Lys Gln Pro Asp Leu Asn Trp Glu Asn Pro Lys
                   165                 170                 175
   Val Arg Arg Glu Val Tyr Glu Met Met Lys Phe Trp Leu Asp Lys Gly
               180                 185                 190
   Val Asp Gly Phe Arg Met Asp Val Ile Asn Met Ile Ser Lys Val Pro
           195                 200                 205
   Glu Leu Pro Asp Gly Glu Pro Gln Ser Gly Lys Lys Tyr Ala Ser Gly
       210                 215                 220
   Ser Arg Tyr Tyr Met Asn Gly Pro Arg Val His Glu Phe Leu Gln Glu
   225                 230                 235                 240
   Met Asn Arg Glu Val Leu Ser Lys Tyr Asp Ile Met Thr Val Gly Glu
                   245                 250                 255
   Thr Pro Gly Val Thr Pro Lys Glu Gly Ile Leu Tyr Thr Asp Pro Ser
               260                 265                 270
   Arg Arg Glu Leu Asn Met Val Phe Gln Phe Glu His Met Asp Leu Asp
           275                 280                 285
   Ser Gly Pro Gly Gly Lys Trp Asp Ile Arg Pro Trp Ser Leu Ala Asp
       290                 295                 300
   Leu Lys Lys Thr Met Thr Lys Trp Gln Lys Glu Leu Glu Gly Lys Gly
   305                 310                 315                 320
   Trp Asn Ser Leu Tyr Leu Asn Asn His Asp Gln Pro Arg Ala Val Ser
                   325                 330                 335
   Arg Phe Gly Asp Asp Gly Lys Tyr Arg Val Glu Ser Ala Lys Met Leu
               340                 345                 350
   Ala Thr Phe Leu His Met Met Gln Gly Thr Pro Tyr Ile Tyr Gln Gly
           355                 360                 365
   Glu Glu Ile Gly Met Thr Asn Val Arg Phe Pro Ser Ile Glu Asp Tyr
       370                 375                 380
   Arg Asp Ile Glu Thr Leu Asn Met Tyr Lys Glu Arg Val Glu Glu Tyr
   385                 390                 395                 400
   Gly Glu Asp Pro Gln Glu Val Met Glu Lys Ile Tyr Tyr Lys Gly Arg
                   405                 410                 415
   Asp Asn Ala Arg Thr Pro Met Gln Trp Asp Asp Ser Glu Asn Ala Gly
               420                 425                 430
   Phe Thr Ala Gly Thr Pro Trp Ile Pro Val Asn Pro Asn Tyr Lys Glu
           435                 440                 445
   Ile Asn Val Lys Ala Ala Leu Glu Asp Pro Asn Ser Val Phe His Tyr
       450                 455                 460
   Tyr Lys Lys Leu Ile Gln Leu Arg Lys Gln His Asp Ile Ile Val Tyr
   465                 470                 475                 480
   Gly Thr Tyr Asp Leu Ile Leu Glu Asp Asp Pro Tyr Ile Tyr Ala Tyr
                   485                 490                 495
   Thr Arg Thr Leu Gly Asn Glu Gln Leu Ile Val Ile Thr Asn Phe Ser
               500                 505                 510
   Glu Lys Thr Pro Val Phe Arg Leu Pro Asp His Ile Ile Tyr Lys Thr
           515                 520                 525
   Lys Glu Leu Leu Ile Ser Asn Tyr Asp Val Asp Glu Ala Glu Glu Leu
       530                 535                 540
   Lys Glu Ile Arg Leu Arg Pro Trp Glu Ala Arg Val Tyr Lys Ile Arg
   545                 550                 555                 560
   Leu Pro